# EUROPEAN PATENT SPECIFICATION

(11) **EP 0 802 778 B1**
(45) Date of publication and mention of the grant of the patent: **02.06.1999**
(21) Application number: 96902145.0
(22) Date of filing: 02.01.1996
(51) Int. Cl.: A61F 13/15

(54) **DISPOSABLE DIAPER**
EINWEGWINDEL
COUCHE JETABLE

(30) Priority: 11.01.1995 JP 18670/95
(43) Date of publication of application: 29.10.1997
(73) Proprietor: THE PROCTER & GAMBLE COMPANY, Cincinnati, Ohio 45202 (US)
(72) Inventor: HEKI, Yukio, Ashiya, Hyogo 659 (JP)
(74) Representative: Hirsch, Uwe Thomas
(86) International application number: US9600529
(87) International publication number: WO9621412

(56) References cited:
- EP-A- 0 323 040
- WO-A-92/22274
- WO-A-94/07450
- WO-A-94/28841

## Description

### BACKGROUND OF THE INVENTION

### Field of the Invention

The present invention relates to a disposable diaper having an absorbent part, a pair of ear parts projecting in opposite directions from the side edges of one longitudinal end portion of the absorbent part, and two fastening means provided on the side edges of the ear parts, respectively, and capable of effectively distributing tensile forces applied to the absorbent part by the fastening means around the waist and around the legs to prevent the leakage of liquid excrements through spaces between the edges of the disposable diaper and the wearer's waist and legs and of giving comfort to the wearer.

### Description of the Related Art

A generally known disposable diaper comprises an absorbent part for covering the wearer's crotch, formed by sandwiching an absorbent core between a top sheet and a back sheet, a pair of ear parts projecting in opposite directions from the side edges of one longitudinal end portion of the absorbent part so as to lap around the wearer's waist, and two fastening means provided on the side edges of the ear parts, respectively. When using this disposable diaper, the disposable diaper is put on the wearer in an ordinary manner, and then the fastening means are attached to por tions of the absorbent part lapping around the waist, on the opposite sides of the fastening means, respectively, to hold the disposable diaper on the wearer. When thus put on the wearer, the disposable diaper must fit to the wearer's waist and legs so that any spaces through which liquid excrements will leak may not be formed between the disposable diaper and the wearer's waist and legs. When putting the dispos able diaper provided with the fastening means provided on the side edges of the ear parts, respectively, on the wearer, it is difficult to concentrate the tensile forces applied to the absorbent part by the fastening means effec tively on portions of the absorbent part lapped around the wearer's waist and legs; that is, spaces are formed around the wearer's legs and liquid excrements leak outside through the spaces around the legs if the disposable diaper is fitted to the wearer's waist, or spaces are formed around the wearer's waist and the disposable diaper cannot lap fitly around the wearer's waist and liquid excrements leak outside through the spaces around the waist if the dispos able diaper is fitted to the wearer's legs.

Various improvements have been proposed to solve such problems and to improve the fit of a disposable diaper to the wearer's waist and legs. Techniques for improving the fit of a disposable diaper to both the waist and the legs are disclosed in U.S. Pat. No. 4,680,030 to Coates, et al., U.S. Pat. No. 4,826,499 to Ahr and U.S. Pat. No. 4,937,887 to Schreiner, which use two pairs of fastening means at tached to a pair of ear parts projecting in opposite direc tions from a portion of the disposable diaper to be lapped around the waist, respectively. These prior art disposable diapers, however, require troublesome work for handling the tow pairs of fastening means and there is room for improve ment in those prior art disposable diapers. Techniques eliminated such a disadvantage are disclosed in U.S. Pat. No. 4,911,702 to O'Leary and U.S. Pat. No. 4,857067 to Wood, which use two fastening means attached to two ear parts, respectively, and capable of effectively concentrating the tensile forces applied thereto on portions of the absorbent part lapped around the waist and the legs. However, these techniques mention nothing about the positions of the two fastening means to distribute the tensile forces applied to the two fastening means effectively and directly around the waist and the legs and about the positions of the two fastening means to distribute the tensile forces applied to the two fastening means around the waist and the legs at an intentionally determined distribution ratio.

### SUMMARY OF THE INVENTION

Accordingly, it is a first object of the present invention to provide a disposable diaper having an absorbent part provided with fastening means positioned so that tensile forces applied thereto are distributed at a desired distribution ratio to a waist lapping section of the absor bent part lapped around the waist and to leg lapping sec tions of the same lapped around the legs.

A second object of the present invention is to provide a disposable diaper having an absorbent part having leg lapping sections to be lapped around the legs, provided with elastic leg fastening members, respectively, and fastening means disposed so that the tensile forces applied thereto may be effectively and directly concentrated on a waist lapping section of the absorbent part lapped around the waist and on the elastic leg fastening members.

A third object of the present invention is to provide a disposable diaper having an absorbent part having a waist lapping section provided with elastic waist fastening members, and leg lapping sections provided with elastic leg fastening members and fastening means positioned so that the tensile forces applied thereto may be effectively and directly concentrated on the elastic leg fastening members and the elastic waist fastening members.

In a first aspect of the present invention, a disposable diaper comprises: an absorbent part formed by sandwiching an absorbent core between a top sheet and a back sheet so that the absorbent core may corresponds to the wearer's crotch when the disposable diaper is put on the wearer; a pair of ear parts projecting in opposite direc tions from the opposite side edges of one longitudinal end portion of the absorbent part, respectively; and two fasten ing means attached to the side edges of the ear parts, respectively. In this disposable diaper, each ear part has a stress relaxing structure, in which a tensile stress smaller than that which is induced in the peripheral portion of the ear part is induced, in a portion thereof other than the peripheral portion, characterized in that each fastening means is attached to the ear part in a pulling section of the side edge of the ear part, overlapping at least part of a first side edge section extending on the side of one longitudinal end edge of the absorbent part corresponding to the ear part from a first boundary line extending from a point on the one longitudinal end edge so as to be tangent to the stress relaxing structure, and part of a second side edge section extending on the side of the transverse center axis of the absorbent part perpendicular to the longitudinal center axis of the absorbent part from a second boundary line extending from a point on the side edge of the absorbent part so as to be tangent to the stress relaxing structure.

The degree of overlap of the pulling section with the first side edge section and the degree of overlap of the same with the second side edge section can be optionally determined. Those degrees of overlap may be different from or equal to each other, the former may be greater than the latter or the latter may be greater than the former. Each fastening means may be attached to the ear part at an angle to the side edge of the ear part.

In a second aspect of the present invention, a dispos able diaper comprises; an absorbent part formed by sandwich ing an absorbent core between a top sheet and a back sheet so that the absorbent core may correspond to the wearer's crotch when the disposable diaper is put on the wearer, and having a first waist lapping section and a second waist lapping section contiguous with the first and the second longitudinal end edge, respectively, a crotch lapping section extending between the first and the second waist lapping section and around the transverse center axis of the absorbent part; elastic leg fastening means extended along the opposite longitudinal side edges of the crotch lapping section of the absorbent part, respectively; a pair of ear parts projecting from the opposite side edges of the first waist lapping section of the absorbent part; and two fasten ing means attached to the side edges of the ear parts, respectively. In this disposable diaper, each ear part has a stress relaxing structure, in which a tensile stress smaller than that which is induced in the peripheral portion of the ear part is induced, in a portion thereof other than the peripheral portion, a portion of the first waist lapping section, extending on the side of the first longitudinal end edge from a first boundary line extending from a point on the first longitudinal end edge so as to be tangent to the stress relaxing structure is a waist lapping component force distributing region to which a component of a tensile force applied to the fastening means acts directly, a section of the side edge of the ear part corresponding to the waist lapping component force distributing region is a first side edge section, a portion of the first waist lapping section, extending on the side of the crotch portion from a second boundary line extending from one end of the elastic leg fastening means on the side of the first waist lapping section so as to be tangent to the stress relaxing structure is a leg lapping component force distributing region to which a component of a tensile force applied to the fasten ing means acts directly, a section of the side edge of the ear part corresponding to the leg lapping component force distributing region is a second side edge section, and each fastening means is attached to the ear part in a pulling section of the side edge of the ear part, overlapping at least part of the first side edge section and part of the second side edge section.

In a third aspect of the present invention, a dispos able diaper comprises; an absorbent part formed by sandwich ing an absorbent core between a top sheet and a back sheet so that the absorbent core may correspond to the wearer's crotch when the disposable diaper is put on the wearer, and having a first waist lapping section and a second waist lapping section contiguous with the first and the second longitudinal end edge, respectively, a crotch lapping section extending between the first and the second waist lapping section around the transverse center axis of the absorbent part; elastic waist fastening means extended along the first longitudinal end edge of the first waist lapping section; a pair of ear parts projecting from the opposite side edges of the first waist lapping section of the absor bent part; and two fastening means attached to the side edges of the ear parts, respectively. In this disposable diaper, each ear part has a stress relaxing structure, in which a tensile stress smaller than that which is induced in the peripheral portion of the ear part is induced, in a portion thereof other than the peripheral portion, a portion of the first waist lapping section, extending on the side of the first longitudinal end edge from a first boundary line extending from one end of the elastic waist fastening means on the side of the ear part so as to be tangent to the stress relaxing structure is a waist lapping component force distributing region to which a component of the tensile force applied to the fastening means acts directly, a section of the side edge of the ear part corresponding to the waist lapping component force distributing region is a first side edge section, a portion of the first waist lapping section, extending on the side of the crotch lapping section from a second boundary line extending from position where the lower end of the ear part joins to the longitudi nal side edge of the crotch lapping section so as to be tangent to the stress relaxing structure is a leg lapping component force distributing region to which a component of the tensile force applied to the fastening means acts directly, a section of the side edge of the ear part cone sponding to the leg lapping component force distributing portion is a second side edge section, and the fastening means is attached to the ear part in a pulling section of the side edge, overlapping at least part of the first side edge section and part of the second side edge section.

The stress relaxing structure of the ear part, formed in a portion other than the peripheral portion of the ear part, in which a tensile stress smaller than that which is induced in the peripheral portion, absorbs the tensile force applied thereto through the fastening means, and the periph eral portion of the ear part transmits the tensile force directly to the absorbent part, so that the tensile force applied to the fastening means can be effectively and directly applied to the waist lapping section and the leg lapping section of the absorbent part.

Attachment of the fastening means to the side edge of the ear part in the pulling section overlapping at least part of the first side edge section and the second side edge section defined in positional connection with the absorbent part and the stress relaxing structure of the ear part ensures the application of the tensile force applied to the fastening means to part of the first longitudinal end edge of the absorbent part on the side of the first waist lapping section or to part of the edge of the ear part on the side of the transverse center axis of the disposable diaper.

The tensile force that acts on part of the longitudinal end edge of the absorbent part on the side of the first waist lapping section or on part of the edge of the ear part on the side of the transverse center axis of the disposable diaper can be adjusted by adjusting the degree of overlap of the pulling section in which the fastening means is attached to the ear part with the first side edge section or the second side edge section. A major part of the tensile force applied to the fastening means is applied to the first waist lapping section when the degree of overlap of the pulling section with the first side edge section is greater than that with the second side edge section, and to a portion of the end edge of the ear part on the side of the transverse center axis of the disposable diaper when degree of overlap of the pulling section with the first side edge section is smaller than that with the second side edge section.

The distribution of the tensile force can be adjusted by selectively determining the angle of the fastening means to the side edge of the ear part. When the fastening means is inclined obliquely upward, a major part of the tensile force applied to the fastening means is distributed to the leg lapping section of the disposable diaper. When the fastening means is declined obliquely downward, a major part of the tensile force applied to the fastening means is distributed to the first waist lapping section.

Determination of the first side edge section in connec tion with the end of the elastic waist fastening means and that of the second side edge section in connection with the end of the elastic leg fastening means ensures the distribu tion of the tensile force applied to the fastening means to the elastic waist fastening means and the elastic leg fastening means.

The above and other objects, features and advantages of the present invention will become more apparent from the following description taken in connection with the accompa nying drawings.

### BRIEF DESCRIPTION OF THE DRAWINGS

Fig. 1 is a development of a disposable diaper in accordance with the present invention;
Fig. 2 is a fragmentary sectional view of a absorbent part of a disposable diaper in accordance with the present invention;
Fig. 3 is a fragmentary sectional view of a ear part; Fig. 4 is a plan view of a stress relaxing structure in a first example included in a disposable diaper in accor dance with the present invention;
Fig. 5 is a stress relaxing structure in a second example included in a disposable diaper in accordance with the present invention;
Fig. 6 is a stress relaxing structure in a third example included in a disposable diaper in accordance with the present invention;
Fig. 7 is a stress relaxing structure in a fourth example included in a disposable diaper in accordance with the present invention;
Fig. 8 is a typical side view of assistance in explain ing a stress relaxing structure forming device;
Fig. 9 is a plan view of a forming plate of the stress relaxing structure forming device;
Fig. 10 is a fragmentary perspective view of a stress relaxing structure formed by the stress relaxing structure forming device of Fig. 8;
Fig. 11 is a fragmentary perspective view of a formed intermediate film shown in Fig. 10, in a half stretched state;
Fig. 12 is a fragmentary perspective view of the formed intermediate film shown in Fig. 10 in a fully stretched state;
Fig. 13 is a fragmentary development of a disposable diaper in accordance with the present invention, of assis tance in explaining the substantially equal distribution of a tensile force applied to a fastener to a waist lapping section and a leg lapping section;
Fig. 14 is a fragmentary development of a disposable diaper in accordance with the present invention, of assis tance in explaining the distribution of a major part and a minor part of a tensile force applied to a fastener to a waist lapping section and a leg lapping section, respective ly, of the disposable diaper;
Fig. 15 is a fragmentary development of a disposable diaper in accordance with the present invention, of assis tance in explaining the distribution of a minor part and a major part of a tensile force applied to a fastener to a waist lapping section and a leg lapping section, respective ly, of the disposable diaper;
Fig. 16 is a fragmentary development of assistance in explaining a first side edge section a and a second side edge section b;
Fig. 17 is a fragmentary development of assistance in explaining the distribution of a tensile force applied to the fastening means;
Fig. 18 is a fragmentary development of assistance in explaining a method of determining a first side edge sec tion;
Fig. 19 is a fragmentary development of assistance in explaining another method of determining a first side edge section;
Fig. 20 is a fragmentary development showing a fasten ing means attached to an ear part in a pulling section equally overlapping a first side edge section a and a second side edge section b;
Fig. 21 is a fragmentary development showing a fasten ing means attached to an ear part in a pulling section with its longitudinal axis aligned with the bisector of the angle between a first boundary line and a second boundary line;;
Fig. 22 is a fragmentary development showing a fasten ing means attached to an ear part in a pulling section having a minor portion overlapping a second side edge section b and a major portion overlapping a first side edge section a
Fig. 23 is a fragmentary development showing a fastening member attached to an ear part in a pulling section having a major portion overlapping a second side edge section b and a minor portion overlapping a first side edge section a;
Fig. 24 is a fragmentary development of assistance in explaining the relation between a stress relaxing structure, and a first side edge section a and a second side edge section b;
Fig. 25 is a fragmentary development of assistance in explaining the relation between a stress relaxing structure, and a first side edge section a and a second side edge section b
Fig. 26 is fragmentary development of a disposable diaper in accordance with the present invention showing a modification of the ear part, and a first side edge section a and a second side edge section b on the ear part;
Fig. 27 is a fragmentary development of a disposable diaper in an embodiment according to the present invention provided with a fastening means extended obliquely upward from a position corresponding to that of the fastening means shown in Fig. 20;
Fig. 28 is a fragmentary development of a disposable diaper in an embodiment according to the present invention provided with a fastening means extended obliquely upward from a position corresponding to that of the fastening means shown in Fig. 23;
Fig. 29 is a fragmentary development of a disposable diaper in an embodiment according to the present invention provided with a fastening means extended obliquely downward from a position corresponding to that of the fastening means shown in Fig. 20;
Fig. 30 is a fragmentary development of a disposable diaper in an embodiment according to the present invention provided with a fastening means extended obliquely downward from a position corresponding to that of the fastening means shown in Fig. 23;
Fig. 31 is a fragmentary development of a disposable diaper, of assistance in explaining a method of defining a second edge section;
Fig. 32 is a fragmentary development of a disposable diaper, of assistance in explaining a method of defining a first side edge section;
Fig. 33 is a fragmentary development of a disposable diaper in accordance with the present invention provided with a fastening means in a modification obliquely attached to an ear part; and
Fig. 34 is a fragmentary development of a disposable diaper in accordance with the present invention provided with a fastening means in a modification obliquely attached to an ear part.

### DESCRIPTION OF THE PREFERRED EMBODIMENTS

In the following description, terms expressing positions, directions and the like indicate those as viewed in the drawings.

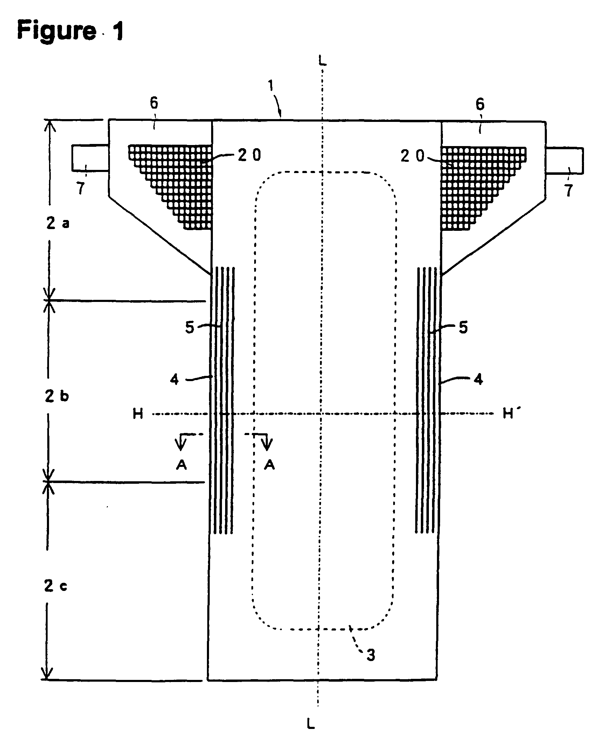

Referring to Fig. 1 a disposable diaper 1 in a first embodiment according to the present invention has a first waist lapping section 2a, a crotch lapping section 2b and a second waist lapping section 2c, and comprises an absorbent part formed substantially in a rectangular shape, an hour glass shape, a T-shape, an asymmetric shape or such, side flaps 4 formed in the opposite side portions of the absor bent part 3, respectively, elastic leg fastening members 5 longitudinally extended in the flaps 4 to provide the flaps 4 with elasticity, a pair of ear parts 6 projecting in opposite directions from the opposite side edges of the upper end portion of the first waist lapping section 2a of the absorbing part 3, respectively, and two fasteners 7 attached to the side edges 6a of the ear parts 6, respectively.

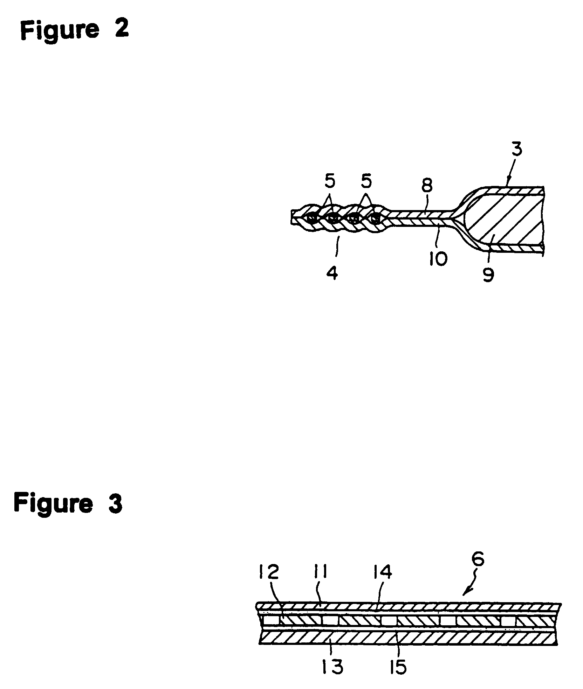

Referring to Fig. 2 showing a sectional view taken on line A-A in Fig. 1, the absorbent part 3 is formed by sandwiching an absorbent core 9 between a liquid-permeable top sheet 8 and a liquid-impermeable back sheet 10. The top sheet 8 and the back sheet 10 have a length and a width greater than those of the absorbent core 9. At least portions of the top sheet 8 and the back sheet 10, corre sponding to the crotch lapping section 2b and extending outward beyond each longitudinal side edge of the absorbent core 9 form a side flap 4, and elastic leg fastening members 5 are extended longitudinally in the side flap 4 to provide the side flap with elasticity. The absorbent core 9 is unirritative to the skin and capable of absorbing and holding liquid excrements including urine. Generally, the absorbent core 9 is formed of crushed wood pulp generally cold cotton pulp. The absorbent core 9 may be formed in any suitable shape and construction as occasion demands. The total absorption of the absorbent core 9 must correspond to a design charge and a desired use. The size and the absorb ing ability of the absorbent core 9 are dependent on the wearer's age.

The back sheet 10 prevents wetting an article, such as a bed sheet or underwear, that comes into contact with the disposable diaper 1 with excrements absorbed and held by the absorbent core 9. The back sheet 10 is a film of a thermoplastic polymer, such as a polyethylene film, a polypropylene film or the like, or a film of a composite material, such as film-coated nonwoven fabric. Preferably, the back sheet 10 is an embossed thermoplastic film simulat ing fabric. The top sheet 8 is is a porous foam sheet, a meshed foam sheet, a perforated plastic film or a woven or nonwoven fabric of a natural textile material, such as wood fibers or cotton fibers, a synthetic textile material, such as polyester fibers or polypropylene fibers, or a blended textile material, such as a blend of a natural textile material (or natural textile materials) and a synthetic textile material (or synthetic textile materials). Prefera bly, the top sheet 8 is formed of a hydrophobic material to separate the liquid absorbed by the absorbent core 9 from the wearer's skin.

The ear parts 6 having a substantially trapezoidal shape are joined to the opposite side edges of the first waist lapping section 2a of the rectangular absorbent part 3, respectively, by a known method, such as heat-sealing or adhesive bonding. The ear parts 6 may be formed integrally with the absorbent part 3 by projecting the respective upper side portions of the top sheet 8 and the back sheet 10 outward in lugs having the shape of the ear parts 6, and directly joining together the projecting lugs of the top sheet 8 and the back sheet 10. The absorbent part 3 and the ear parts 6 may be formed in an integral structure by cutting a laminated sheet formed by sandwiching a sheet between the top sheet 8 and the back sheet 10 in desired dimensions. Preferably, the ear parts 6 are formed by laminating a nonwoven fabric 11 of natural fibers, synthetic fibers or a blend of natural fibers and synthetic fibers, a thermoplastic film 13 of polyethylene or polypropylene, and a porous film 12 formed by a known process and sandwiched between the nonwoven fabric 11 and the thermoplastic film 13 by adhesive layers 14 and 15 as shown in Fig. 3. When each of the ear parts 6 is formed of an extension of the top sheet 8 and an extension of the back sheet 10, the nonwoven fabric 11 and the thermoplastic film 13 correspond to the extension of the top sheet 8 and the extension of the back sheet 10, respectively. The porous film 12 sandwiched between the nonwoven fabric 11 and the thermoplastic film 13 provides the ear parts 6 with firmness, which facilitates handling the ear parts 6 when putting the disposable diaper 1 on the wearer.

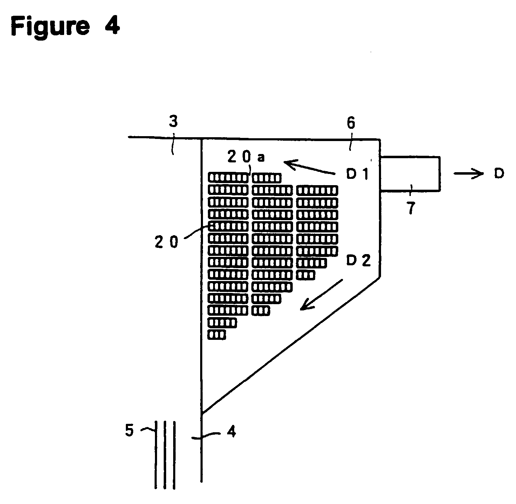

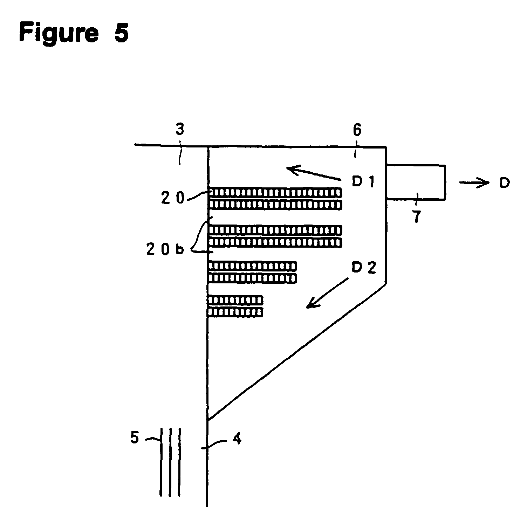

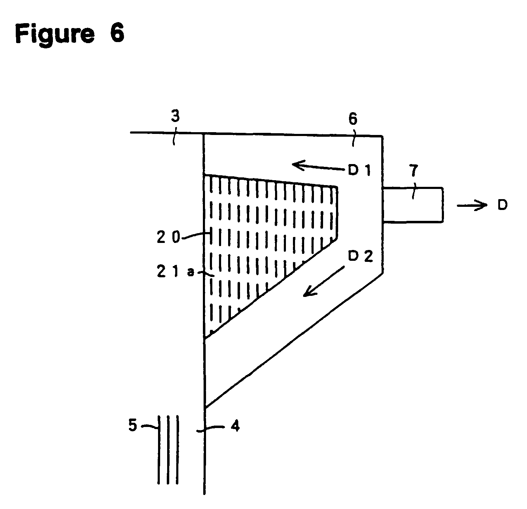

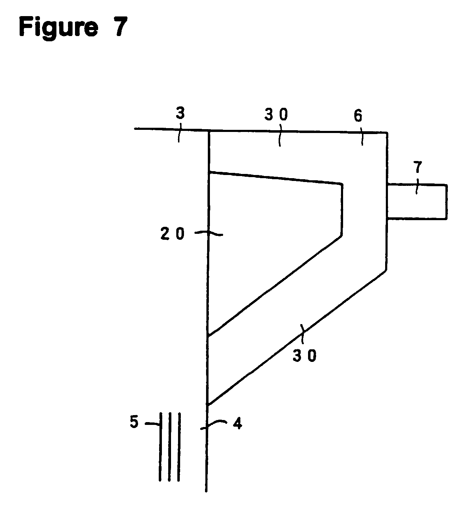

As shown in Fig. 1, the ear parts 6 have each a stress relaxing structure 20 of a shape similar to that of the ear part 6 in their inner portions excluding the peripheral portions. As shown in Fig. 1, the stress relaxing structure 20 may be formed in a trapezoidal shape similar to and smaller than that of the ear part 6. The stress relaxing structure 20 may be formed in a substantially trapezoidal shape having longitudinal gaps 20a as shown in Fig. 4 or in a substantially trapezoidal shape having transverse gaps 20b as shown in Fig. 5. The stress relaxing structure 20 may be formed in any suitable shape other than a trapezoidal shape, such as a triangular shape, a rectangular shape, a shape of a part of an ellipse or a shape of a part of a circle, provided that the stress relaxing structure 20 has a func tion to relax stress. The stress relaxing structure 20 of the ear part 6 may be an aggregate of a plurality of longi tudinal slits 21a formed in the laminated sheet forming the ear part 6 as shown in Fig. 6. The stress relaxing struc ture 20 may be a single slit 21a provided that the single slit 21a is capable of intercepting the transmission of a tensile force applied to the ear part 6 to the absorbent part 3. As shown in Fig. 7, the ear part 6 may be formed in an elastic structure, and a strip of an unstretchable film or an unstretchable strand 30 may be attached to a peripher al portion of the elastic structure excluding an inner trapezoidal elastic portion corresponding to the stress relaxing structure 20 to use the inner trapezoidal elastic portion as the stress relaxing structure 20.

When a tensile force is applied to the ear part 6, a tensile stress induced in the stress relaxing structure 20 is smaller than that which is induced in the peripheral portion of the ear part 6 surrounding the stress relaxing structure 20. Tensile stress is a resistive force developed by a material bearing a tensile load. Therefore, "a struc ture having a small tensile stress" is a structure yielding to and easily stretchable by a comparatively small tensile force. Structures expressed by the term "stress relaxing structure" include a vacancy formed in the ear part 6 and virtually serving as a stress relaxing structure 20.

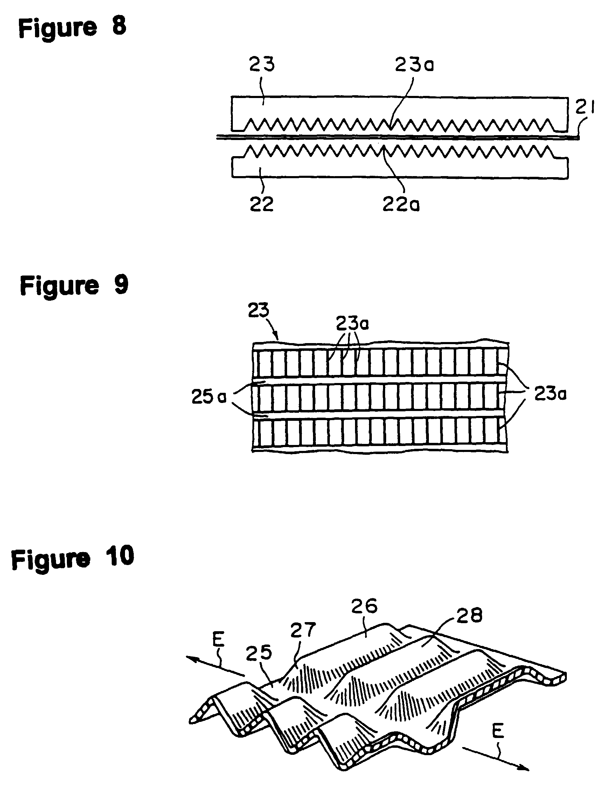

Preferably, the stress relaxing structure 20 is formed by the following method. As shown in Fig. 8, a sheet 21 to form the ear part 6 is compressed between a plate 22 provid ed with a plurality of ridges 22a in an area of the inner surface thereof corresponding to the stress relaxing struc ture 20, and a plate 23 provided with a plurality of ridges 23a in a area of the inner surface thereof corresponding to the stress relaxing structure 20. As shown in Fig. 9, narrow grooves 25a are formed across the plurality of ridges 23a of the plate 23. The plurality of ridges 22a of the plate 22 are continuous and not interrupted. As shown in Fig. 10, the stress relaxing structure 20 formed by thus compressing the sheet 21 between the plates 22 and 23 has a plurality of undeformed sections 25, a plurality of perma nently deformed sections 26, and transitional sections 27 between the undeformed sections 25 and the permanently deformed sections 26. Ridges 28 are formed in the perma nently deformed sections 26. The undeformed sections 25 are defined by the narrow grooves 25a of the plate 23 and the corresponding portions of the ridges 22a of the plate 22, and the permanently deformed sections 26 are defined by the respective ridges 22a and 23a of the plate 22 and the plate 23.

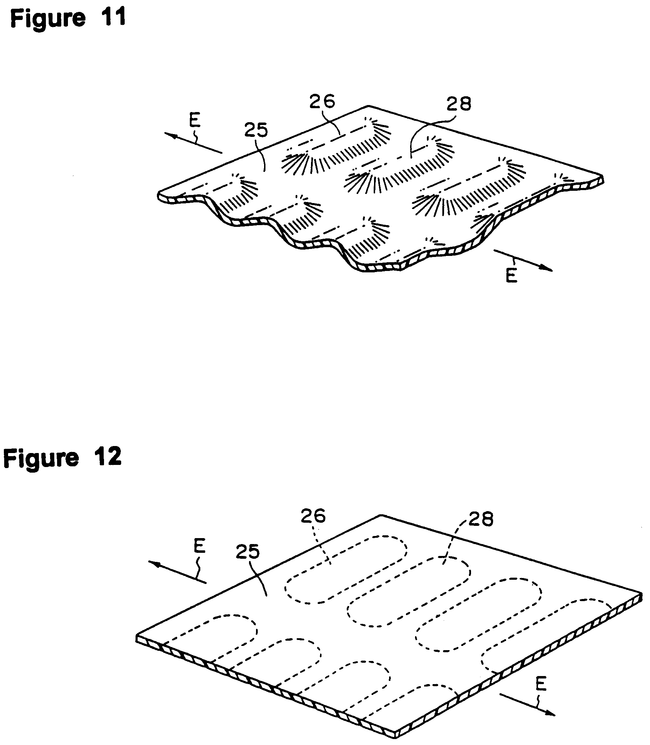

When the ear part 6 is tensioned by forces acting in the directions of the arrows E as shown in Fig. 10, the undeformed sections 25 are plastically stretched to some extent as shown in Fig. 11 and, as the undeformed sections 25 are further stretched, the ridges 28 of the permanently deformed sections 26 are flattened as shown in Fig. 12 and the ear part 6 cannot be stretched any further. When the sheet 21 having such characteristics is a laminated sheet, at least one of the component layer of the sheet 21 is formed of a stretchable film, such as a polyolefin film, a low-density linear polyethylene film, a low-density polyeth ylene film, a high-density polyethylene film or a polypropylene film.

The stress relaxing structure 20 that can be stretched by a small tensile force is thus formed in the sheet 21 forming the ear part 6. Since the periphery of the sheet 21 surrounding the stress relaxing structure 20 is unprocessed, the periphery of the sheet 21 does not yield to tension when the sheet 21 is tensioned or the periphery is harder to stretch than the stress relaxing structure 20. When tensioned, the stress relaxing structure 20 yields to the tensile force to absorb the tensile force and does not cause the peripheral portion of the ear part 6 to shrink.

The sheet 21 forming the ear part 6 may be formed of a somewhat stretchable material. When formed of such a somewhat stretchable material, the stress relaxing structure 20 can be formed in the sheet 21 by the aforesaid process. In either case, the stress relaxing structure 20 can be stretched by a tensile force smaller than that necessary for stretching the peripheral portion of the ear part 6.

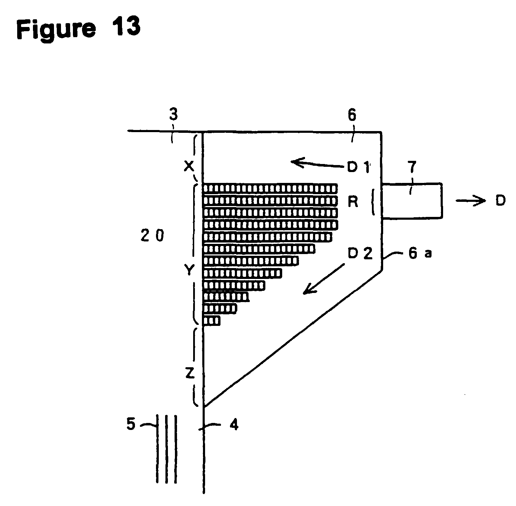

As shown in Fig. 13, when a tensile force D is applied to the fastener 7 in a direction perpendicular to the side edge 6a of the ear part 6, the stress relaxing structure 20 of the ear part 6 serves to divide the tensile force D into a component tensile force D1 acting on the waist lapping portion and a component tensile force D2 acting on the leg lapping portion. That is, since a tensile force greater than that necessary for stretching the stress relaxing structure 20 must be applied to the peripheral portions of the ear part 6 to stretch the peripheral portion, most part of the tensile force D applied to the fastener 7 is divided into the component tensile forces D1 and D2, and the compo nent tensile forces D1 and D2 act directly on regions X and Z of the absorbent part 3, respectively. Even if part of the tensile force D acts on the stress relaxing structure 20, the stress relaxing structure 20 is not stretched in a extreme state as shown in Fig. 12 because the stress relax ing structure 20 can be stretched by a comparatively small tensile force and the peripheral portion surrounding the stress relaxing structure 20 cannot be virtually stretched. Thus, the stretchable stress relaxing structure 20 absorbs the tensile force that acts thereon and, consequently, any tensile force is not transmitted directly to a region Y of the absorbent part 3. Thus the tensile force D applied to the fastener 7 is divided into the component tensile force D1 acting on the waist lapping portion of the absorbent part 3 and the component tensile force D2 acting on the leg lapping portion of the absorbent part 3 to fasten the absorbent part 3 around the waist and the leg.

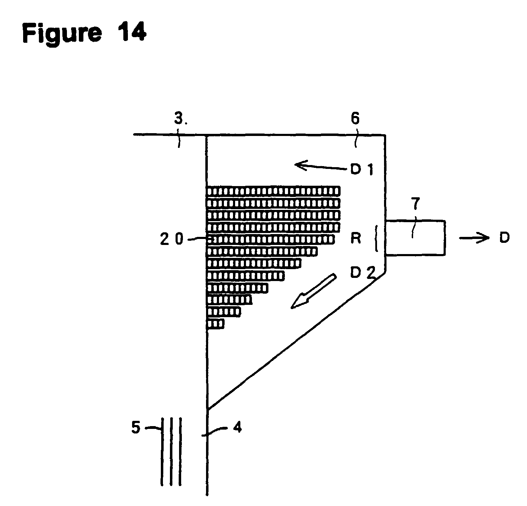

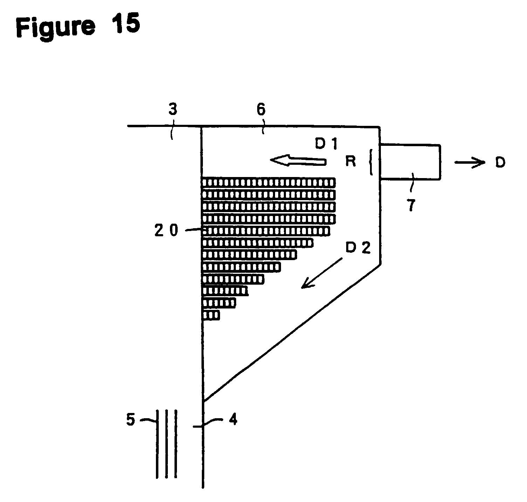

The division of the tensile force D applied to the fastener 7 into the component tensile forces D1 and D2 cam be adjusted by adjusting the position of a pulling section R where the fastener 7 is attached to the oblique side edge 6a of the ear part 6. When the pulling section R corresponds substantially to the middle of the side edge of the stress relaxing structure 20 as shown in Fig. 13, the tensile force D applied to the fastener 7 in the direction of the arrow is divided into the component tensile force D1 acting around the waist and the tensile force D2 acting around the leg, and the component tensile forces D1 and D2 are substantially equal to each other. When the pulling section R is at a position shown in Fig. 14 below the position of the pulling section R shown in Fig. 13, the tensile force D applied to the fastener 7 in the direction of the arrow is divided into the component tensile forces D1 and D2, the component tensile force D1 that acts around the waist is comparatively small and the component tensile force D2 that acts around the leg is comparatively large. When the pulling section R is at a position shown in Fig. 15 above the position of the pulling section R shown in Fig. 13, the tensile force D applied to the fastener 7 in the direction of the arrow is divided into the component tensile forces D1 and D2, the component tensile force D1 that acts around the waist is comparatively large and the component tensile force D2 that acts around the leg is comparatively small.

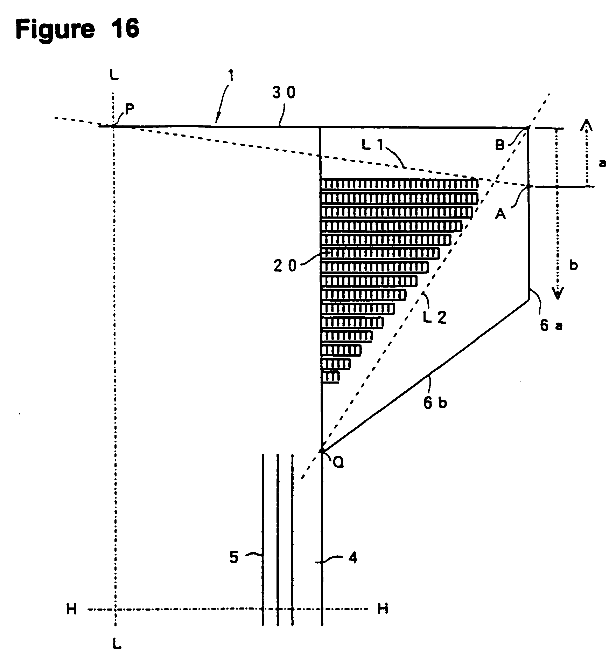

The position of the pulling section R and the position al relation between the same and other parts to distribute the tensile force applied to the fastener 7 surely to desired portions of the absorbent part 3 will be described below. The position of the pulling section R for the fastener 7 on the oblique side edge 6a is determined by construction shown in Fig. 16. The position of the pulling section R is important for effectively and directly distrib uting a tensile force applied to the fastener 7 to a desired waist lapping portion and a desired leg lapping portion of the disposable diaper 1. Referring to Fig. 16, a line L-L' indicates the longitudinal center axis of the disposable diaper 1 (absorbent part 3) and a line H-H' indicates the transverse center axis of the disposable diaper 1 (absorbent part 3) perpendicular to the longitudinal center axis L-L'. Indicated at P is the intersection point of the longitudinal center axis L-L' and the upper edge 1a of the first waist lapping section 2a, at Q is the intersection point of the lower edge 6b of the ear part 6 on the side of the trans verse center axis H-H' and the side flap 4, at L1 is a first boundary line extending from the intersection point P so as to be tangent to a point on the stress relaxing structure 20 on the side of the upper edge 1a, at L2 is a second boundary line extending from the intersection point Q so as to be tangent to a point on the stress relaxing structure 20 on the side of the transverse center axis H-H', at A is the intersection point of the first boundary line L1 and the side edge 6a, and at B is the intersection point of the second boundary he L2 and the side edge 6a. A first side edge section a extends on the side of the upper edge 1a from the intersection point A, and a second side edge section b extends on the side of the transvesse center axis H-H' from the intersection point B.

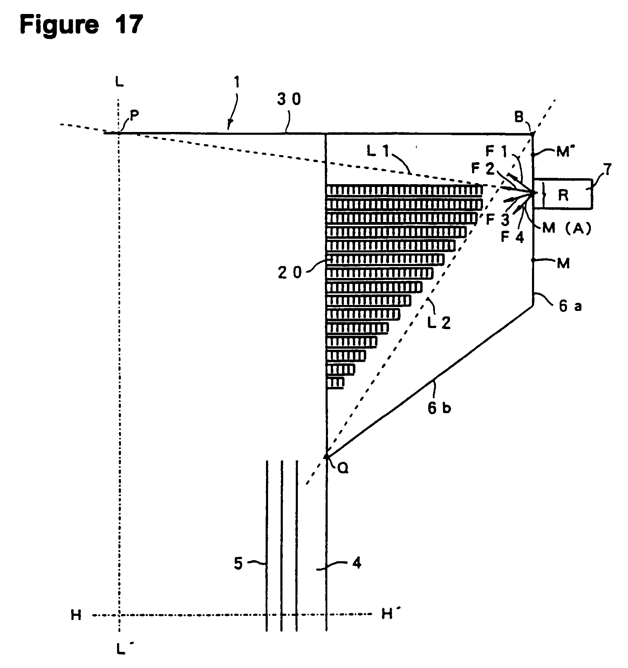

A tensile force D applied to the side edge 6a by the fastener 7 is distributed in the ear part 6 as shown in Fig. 17. In Fig. 17, a point M of action of the tensile force applied to the fastener 7 is in the pulling section R in which the fastener 7 is attached to the side edge 6a and coincides with the intersection point A in this example. When part of a tensile force applied to the fastener 7 acts on the side edge 6a at the point M of action, the part of the tensile force is decomposed into representative compo nent forces F1, F2, F3 and F4. The component force F1 is directed toward the upper edge 1a and not directly toward the intersection point P. Therefore, the component force F1 does not act directly on the intersection point P. The direction of the component force F2 is aligned with the first boundary line L1 and hence the component force F2 acts directly on the intersection point P. The component force F3 is directed toward the stress relaxing structure 20 and tends to stretch the stress relaxing construction 20. However, since the stress relaxing structure 20 absorbs the force applied thereto, the component force F3 is not trans mitted to the region Y of the absorbent part 3 shown in Fig. 13. The component force F4 is directed toward the intersec tion point Q and acts directly on the absorbent part 3 at the intersection point Q.

If the point M of action in the pulling section R in which the fastener 7 is attached to the side edge 6a is on the side of the transverse center axis H-H' with respect to the intersection point A, i.e., at a point M' outside the first side edge section a (Fig. 16), the component force F2 directed from the point M' toward the intersection point P is absorbed by the stress relaxing structure 20 and is ineffective. Therefore, no component force acts directly on the intersection point P if the point M of action of the tensile force applied to the fastener 7 on the side edge 6a is at the point M'. If the point M action is shifted to a point M'' on the side of the upper edge 1a with respect to the intersection point A shown in Fig. 17, i.e., if the point M of action is in the first side edge section a, some component force acts directly on the intersection point P.

As is obvious from the foregoing explanation, the first boundary line L1 is a boundary line between positions for the point M of action that enables the direct action of a component force of the tensile force applied to the fastener 7 on the intersection point P and those for the point M of action that disables the direct action of the component force on the intersection point P. When the point M of action at which the tensile force applied to the fastener 7 acts on the ear part 6 is in the first side edge section a, at least part of the tensile force applied to the point M of action is able to act directly on the intersection point P. The second boundary line L2 drawn in connection with the intersection point Q is a boundary line between positions for the point M of action that enables the direct action of a component force of the tensile force applied to the fastener 7 on the intersection point Q. When the point M of action at which the tensile force applied to the fastener 7 acts on the ear part 6 is in the second side edge section b, at least part of the tensile force applied to the point M of action is able to act directly on the intersection point Q. Accordingly, part of the tensile force applied to the fastener 7 can be applied directly on the desired points P and Q on the absorbent part 3 when the pulling section R in which the fastener 7 is attached to the side edge 6a of the ear part 6 overlaps part of the first side edge section a defined by the first boundary line L1 and part of the second side edge section b defined by the second boundary line L2. Since the intersection point P is on the longitudinal center axis L-L' of the disposable diaper 1, the waist lapping portion of the disposable diaper 1 can be firmly fastened around the waist by applying tensile forces to the two ear parts 6, when part of each of the tensile forces can be applied directly to the intersection point P. When part of the tensile force applied to each fastener 7 can be directly applied to the intersection point Q, the leg lapping portion of the absorbent part 3 can be firmly fastened around the leg.

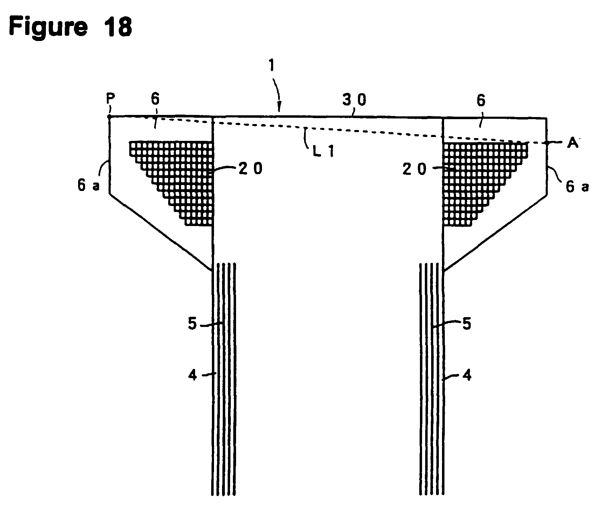

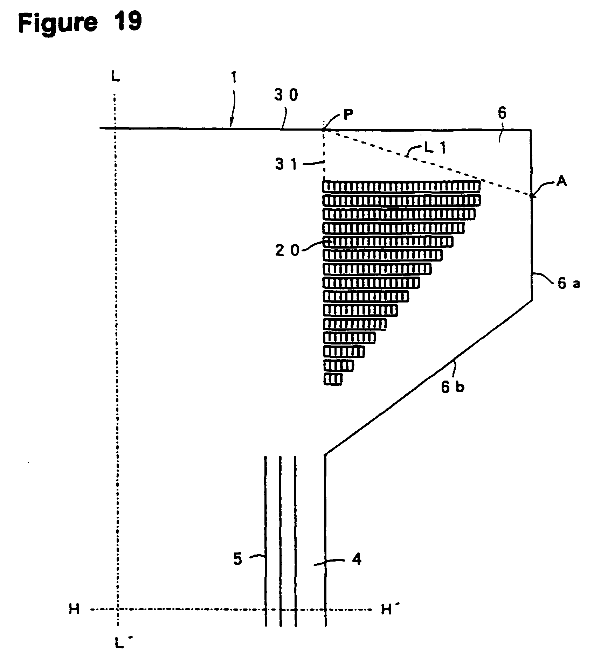

In a disposable diaper 1 in a second embodiment accord ing to the present invention, a first boundary line L1 may be extended from the intersection point P of the leftward extension of the upper edge 1a of a absorbent part 3 and the side edge 6a of a left ear part 6 as shown in Fig. 18. A first boundary line L1 may be extended from the intersection point P of a line extending along the inner side of a stress relaxing structure 20 in parallel to the longitudinal center axis L-L' of a absorbent part 3 and the upper end edge 1a of the absorbent part 3 as shown in Fig. 19. In either case, the first boundary line L1 is extended from the intersection point P in tangent to an upper point on the stress relaxing structure 20, to determine an inter section point A on the side edge 6a of a right ear part 6.

The respective magnitudes of a component tensile force D1 for fastening the waist lapping portion of the absorbent part 3 around the waist and a component tensile force D2 for fastening the leg lapping portion of the absorbent part 3 around the leg are determined by adjusting the ratio of the size of a portion of the pulling section R, in which a fastener 7 is attached to the side edge 6a, overlapping the first side edge section a to the size of the pulling section R, and the ratio of the size of a portion of the pulling section R overlapping the second side edge section b to the size of the pulling section R. The greater the portion of the puffing section R, overlapping the first side edge section a, the greater is the ratio of the component tensile force D1 that acts on the intersection point P to the tensile force applied to the fastener 7. An area defined by the first boundary line L1, the upper edge of the ear part 6 and a portion of the side edge 6a of the ear part 6 extending upward from the intersection point A is a waist lapping portion fastening component force distributing area 42, and a portion of the side edge 6a associated with the waist lapping portion fastening component force distributing area 42 is a first side edge section a. As long as part of the pulling section R in which the fastener 7 is attached to the side edge 6a overlaps the first side edge section a, a component tensile force of the tensile force applied to the fastener 7 acts directly on the intersection point P. The greater the portion of the puffing section R, overlapping the first side edge section a, the greater is the component tensile force D1 distributed to the waist lapping portion fastening component force distributing area 42.

On the other hand, the greater the portion of the pulling section R, overlapping the second side edge section b, the greater is the component tensile force D2 that acts directly on the intersection point Q. An area defined by the second boundary line L2, the lower side edge 6b of the ear part 6 and a portion of the side edge 6a of the ear part 6 extending downward from the intersection point B is a leg lapping portion fastening component force distributing area 43, and a portion of the side edge 6a associated with the leg lapping portion fastening component force distributing area 43 is a second side edge section b. As long as part of the pulling section R overlaps the second side edge section b, a component tensile force of the tensile force applied to the fastener 7 acts directly on the intersection point Q. The greater the portion of the pulling section R, overlap ping the second side edge section b, the greater is the component tensile force D2 distributed to the leg lapping portion fastening component force distributing area 43.

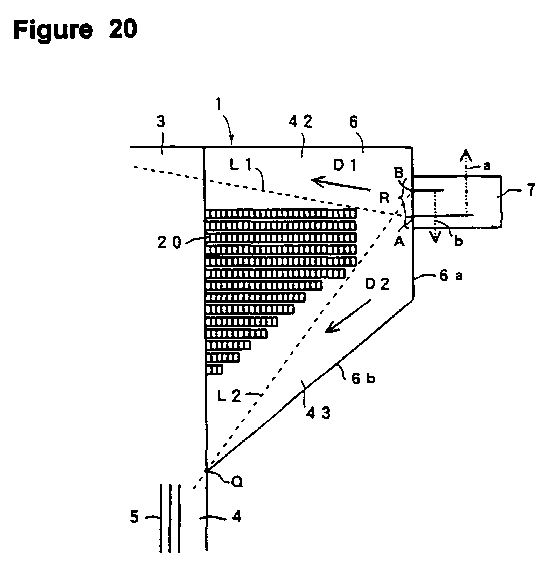

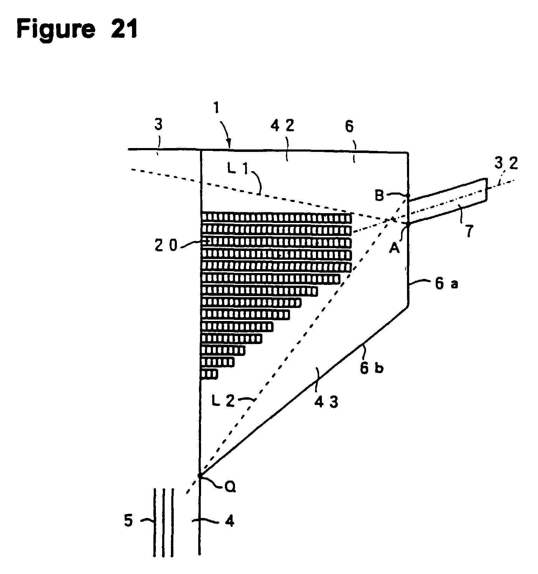

When the pulling section R, in which the fastener 7 is attached to the ear part, includes the intersection points A and B, and the intersection points A and B are at equal distances from the bisector of the pulling section R as shown in Fig. 20, a portion of the pulling section R over lapping the first side edge section a and a portion of the pulling section R overlapping the second side edge section b are substantially equal to each other. Therefore, the component tensile forces D1 and D2 distributed to the waist lapping portion fastening component force distributing area 42 and the leg lapping portion fastening component force distributing area 43 are substantially equal to each other, and hence substantially equal tensile forces are applied to the intersection points P and Q, respectively. When the fastener 7 is attached to the ear part 7 with its longitudi nal axis aligned with the bisector of the angle between the first boundary line L1 and the second boundary line L2 as shown in Fig. 21, the tensile force can be further equally distributed to the component force distributing areas 42 and 43.

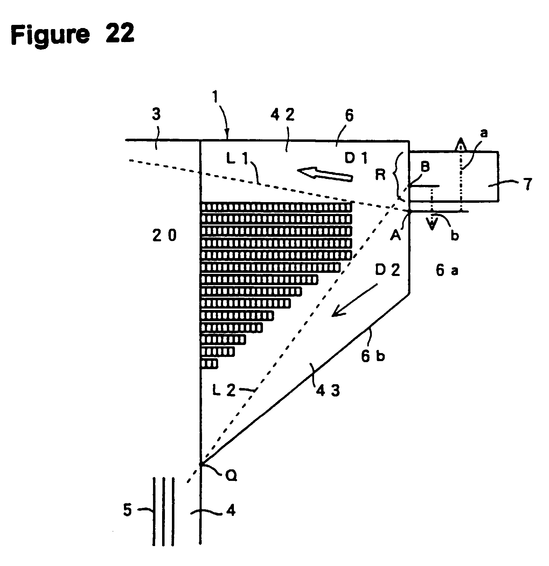

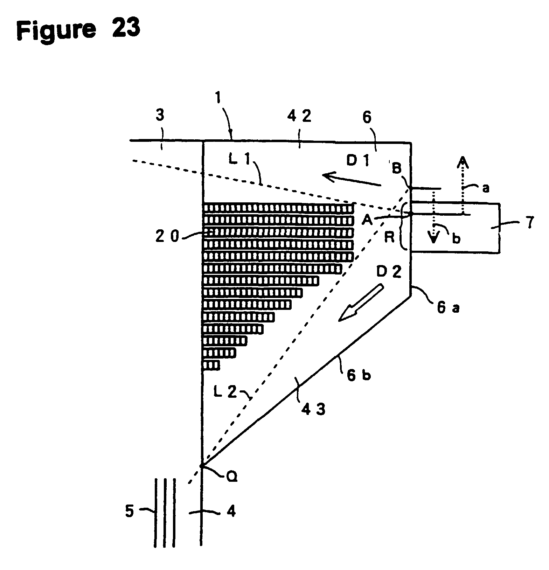

The ratio between the component tensile force D1 distributed to the waist lapping portion fastening component force distributing area 42 and the component tensile force D2 distributed to the leg lapping portion fastening compo nent force distributing area 43 can be adjusted by adjusting the the ratio of the size of a portion of the pulling section R, in which a fastener 7 is attached to the side edge, 6a, overlapping the first side edge section a to the sage of the pulling section R, and the ratio of the size of a portion of the pulling section R overlapping the second side edge section b to the size of the pulling section R. When the pulling section R overlaps the first side edge section a entirely and overlaps the second side edge section b partly as shown in Fig. 22, a major part of the tensile force applied to the fastener 7 is distributed to the waist lapping portion fastening component force distributing area 42. When the pulling section R overlaps the second side edge section b entirely and overlaps the first side edge section a partly as shown in Fig. 23, a major part of the tensile force applied to the fastener 7 is distributed to the leg lapping portion fastening component force distribut ing area 43. The pulling section R need not necessarily be included in either the first side edge section a or the second side edge section b, provided that pulling section R overlaps the first side edge section a and the second side edge section b at different overlapping ratios, respec tively.

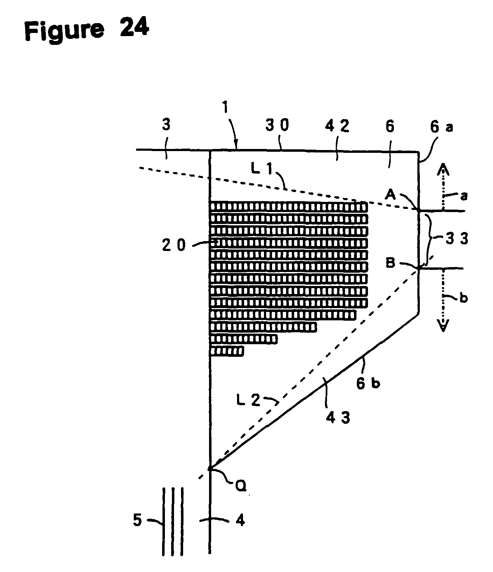

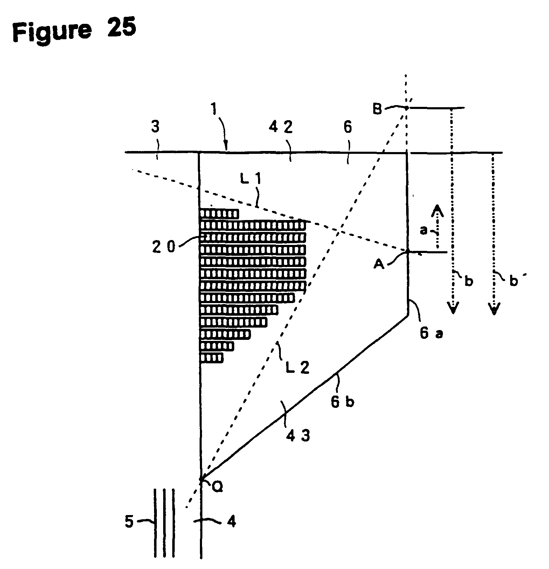

Figs. 24 and 25 show the dependence of the relation between the first side edge section a and the second side edge section b on the position of a stress relaxing structure 20 in an ear part 6 in a disposable diaper 1 in a third embodiment according to the present invention. Referring to Fig. 24, the stress relaxing structure 20 has a shape resembling a rectangle, which is different from the shape of the stress relaxing structure 20 shown in Fig. 16. In the third embodiment, the first boundary line L1 extending from the intersection point P so as to be tangent to the stress relaxing structure 20 and the second boundary line L2 extending from the intersection point Q so as to be tangent to the stress relaxing structure 20 do not intersect each other within the ear part 6. The lines L1 and L2 intersect the side edge 6a at intersection points A and B, respec tively. A first side edge section a extends on the side of the upper edge 1a of an absorbent part 3 from the intersec tion point A, a second side edge section b extends on the side of the transverse center axis H-H' of the absorbent part 3 from the intersection point B, and a neutral section 33 connected with neither the waist lapping portion fasten ing component force distributing area 42 nor the leg portion fastening component force distributing area 43 is formed between the first side edge section a and the second side edge section b. The pulling section R in which a fastener 7 is attached to the side edge 6a must extend across the neutral section 33 and overlap the side edge sections a and b partly to concentrate the component forces of the tensile force applied to the fastener 7 directly on the waist lapping portion and the leg lapping portion; that is, the component forces of the tensile force applied to the fasten er 7 can be directly concentrated on desired portions by determining the position of the pulling section R so that the pulling section R overlap the side edge sections a and b partly.

In a disposable diaper 1 in a fourth embodiment accord ing to the present invention shown in Fig. 25, a first boundary line L1 is extended from the intersection point P so as to be tangent to a stress relaxing structure 20 and intersects the side edge 6a of an ear part 6 at an intersec tion point A, and a second boundary line L2 is extended from the intersection point Q so as to be tangent to the stress relaxing structure 20 and intersects a extension of the side edge 6a at an intersection point B. Since the inter section point B is a virtual point apart from the ear part 6, an actual second side edge section b' is a portion of a second side edge section b, on the side edge 6a. A fastener 7 is attached to the side edge 6a in a pulling section R partially overlapping the first side edge section a and the actual second side edge section b'. Thus the side edge sections a and b can be defined even if the lines L1 and L2 do not intersect the side edge 6a. In this specification, an expression, "a point where the first boundary line L1 (second boundary line L2) intersects the side edge 6a" has an implication that "a point where the first boundary line L1 (second boundary line L2) intersects an extension of the side edge 6a".

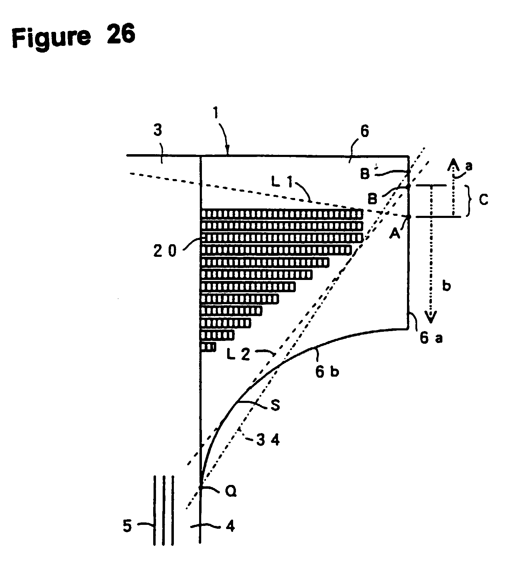

A disposable diaper 1 in a fifth embodiment according to the present invention is provided with ear parts 6 each having a lower edge 6b having a lower curved portion S curved in the shape of a circular arc as shown in Fig. 26. A second boundary line L2 is tangent to the lower curved portion S of the lower edge 6b and a stress relaxing struc ture 20 and intersects the side edge 6a of the ear part 6 at an intersection point B. Therefore, a component tensile force of a tensile force applied to the ear part 6 at the intersection point B, acting along the second boundary line L2 can be transmitted to an absorbent part 3. A line 34 extended from the the intersection point of the lower edge 6b of the ear part 6 and a side flap 4 extended along the side edge of an absorbent part 3 so as to be tangent to the stress relaxing structure 20 intersects the side edge 6a at an intersection point B'. The transmission of a component tensile force of a tensile force applied to the ear part 6 at the intersection point B' and transmitted along the line 34 is intercepted at the intersection point of the line 34 and the lower edge 6b. In this embodiment, a first side edge section a extends upward from the intersection point A, and a second side edge section b extends downward from the intersection point B. A pulling section R in which a fastener 7 is attached to the side edge 6a must overlap at least a section c between the intersection points A and B in which the first side edge section a and the second side edge section b overlap each other.

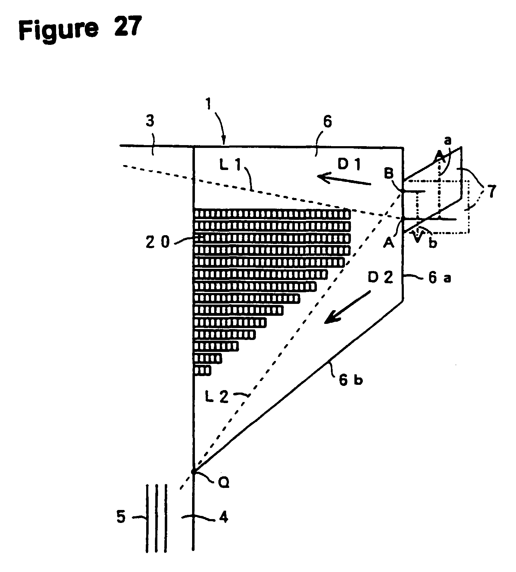

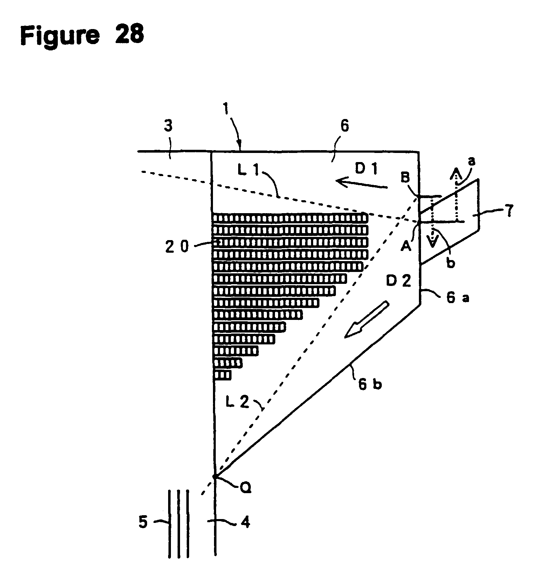

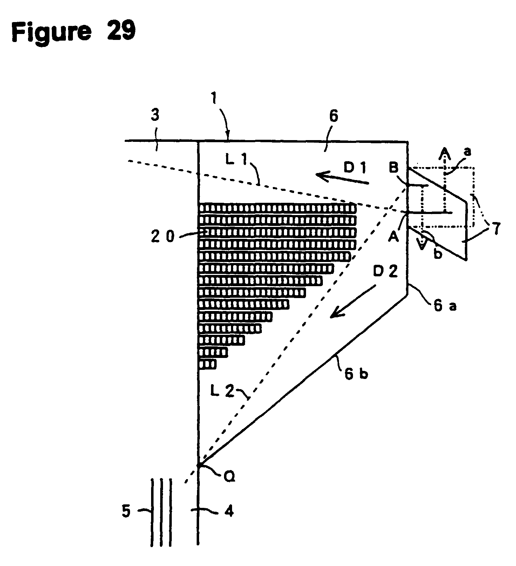

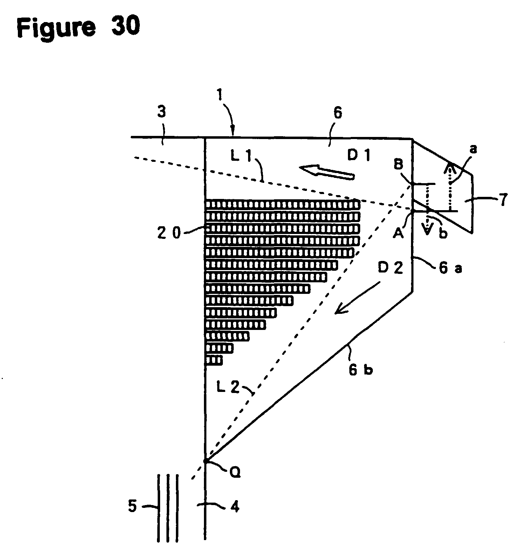

Figs. 27 to 30 are fragmentary developments of dispos able diapers in a sixth to ninth embodiment according to the present invention, which differ from the disposable diaper shown in Fig. 1 only in the direction of extension of the fastener. In each of the disposable diapers 1 of Figs. 27 and 28, a fastener 7 is attached to the side edge 6a of an ear part 6 so as to extend at a inclination_meeting an inequality: 0 <_< 45°, In each of the disposable diapers 1 of Figs. 29 and 30, a fastener 7 is attached to the side edge 6a of an ear part 6 so as to extend at an inclination_meeting an inequality: 0 < _ < 45°.

When a tensile force D is applied to the fastener 7 attached to the side edge 6a of the ear part 6 as shown in Fig. 27 in the direction of extension of the fastener 7, a component tensile force D1 distributed to the waist lapping portion of an absorbent part 3 is smaller than a component tensile force D2 distributed to the leg lapping portion of the absorbent part 3. Since the fastener 7 is attached to the side edge 6a in a pulling section R overlapping both the first side edge section a and the second side edge section b, the tensile force D is distributed to both the waist lapping portion and the leg lapping portion.

In the disposable diaper 1 of Fig. 28, a portion of the pulling section R partly overlapping the second side edge section b is greater than that of the pulling section R partly overlapping the first side edge section a. There fore, the component tensile force D2 distributed to the leg lapping portion is greater than the component tensile force D2 applied to the leg lapping portion of the disposable diaper shown in Fig. 27.

When a tensile force D is applied to the fastener 7 attached to the side edge 6a of the ear part 6 as shown in Fig. 29 in the direction of extension of the fastener 7, a component tensile force D1 distributed to the waist lapping portion of an absorbent part 3 is greater than a component tensile force D2 distributed to the leg lapping portion of the absorbent part 3. Since the pulling section R overlaps both the first side edge section a and the second side edge section b, the component tensile force D2, though smaller than the component tensile force D1, can be distributed to the leg lapping portion.

In the disposable diaper 1 of Fig. 30, a portion of the pulling section R partly overlapping the first side edge section a is greater than that of the pulling section R partly overlapping the second side edge section b. There fore, the component tensile force D1 distributed to the waist lapping portion is greater than the component tensile force D2 applied to the waist lapping portion of the dispos able diaper shown in Fig. 29.

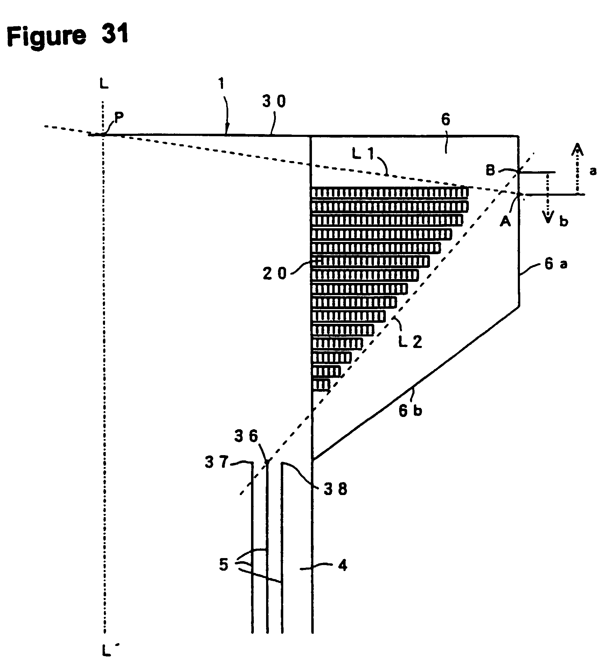

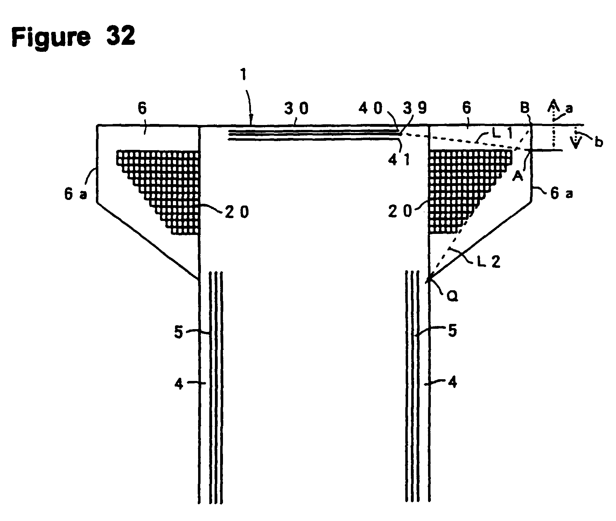

Figs. 31 and 32 show disposable diapers in a tenth and an eleventh embodiment according to the present invention. The disposable diaper 1 shown in Fig. 31 is similar in construction to that shown in Fig. 16, except that a second boundary line L2 in Fig. 31 for defining a second side edge section b is extended from the upper end 36 of the middle elastic leg fastening member 5 so as to be tangent to a stress relaxing structure 20 and intersects the side edge 6a of an ear part 6 at an intersection point B. Accordingly, part of a tensile force applied to the ear part 6 at the intersection point B acts directly on the upper end 36 of the middle elastic leg fastening member 5. The second boundary line L2 may be extended from the upper end 37 of the inner elastic leg fastening member 5 or the upper end 38 of the outer elastic leg fastening member 5. The disposable diaper 1 shown in Fig. 32 is similar in construction to that shown in Fig. 16, except that a first boundary line L1 in Fig. 32 for defining a first side edge section a is extended from the end 39 of the middle elastic waist fastening member 35 so as to be tangent to a stress relaxing structure 20 and intersects the side edge 6a of an ear part 6 at an intersec tion point A. Accordingly, part of a tensile force applied to the ear part 6 at the intersection point A acts directly on the ends 39 of the middle elastic waist fastening member 35. The first boundary line L1 may be extended from the end 40 of the upper elastic waist fastening member 35 or the end 41 of the lower elastic waist fastening member 35.

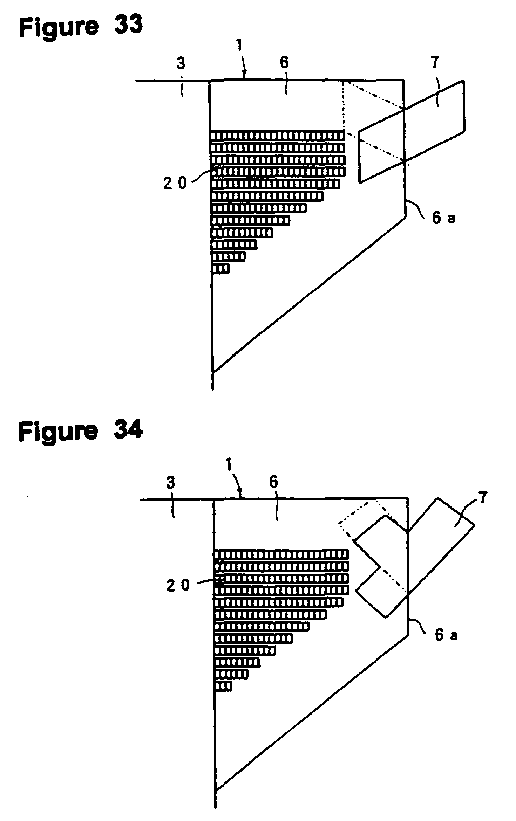

The fastener 7 obliquely attached to the side edge 6a of the ear part 6 may be folded back at the middle thereof as indicated by broken lines as shown in Fig. 33 when packaging the disposable diaper and may project from the side edge 6a when the disposable diaper is unfolded for use. The fastener 7 may be formed in the shape of a fork and may be folded back as indicated by broken lines in Fig. 34. When thus folded back at the middle, no portion of the fastener 7 projects outward from the ear part 6. The fork-shaped fastener 7 shown in Fig. 34 ensures further reliable distribution of a tensile force applied thereto to the waist lapping portion and the leg lapping portion. Since the stress relaxing structure 20 is elastic, the stress relaxing structure 20 conforms easily to the shape of the hipbone and the like, which improves the wearing comfort of the wearer.

## Claims

1. A disposable diaper (1) comprising: an absorbent part (3) formed by sandwiching an absorbent core (9) between a top sheet (8) and a back sheet (10) so that the absorbent core may corresponds to the wearer's crotch when the disposable diaper is put on the wearer, and having a first longitudinal end edge and a second longitudinal end edge; a pair of ear parts (6) projecting in opposite directions from the opposite side edges of one longitudinal end portion of the absorbent part on the side of the first longitudinal end edge, respectively; and two fastening means (7) attached to the side edges of the ear parts, respectively;
each ear part (6) having a stress relaxing structure (20), in which a tensile stress smaller than that which is induced in the peripheral portion of the ear part is induced, in a portion thereof other than the peripheral portion, characterized in that each fastening means attached to the ear part in a pulling section of the side edge of the ear part, overlapping at least part of a first side edge section (a) on the side edge of the ear part, extending on the side of the first longitudi nal end edge of the absorbent part from the intersection point of the side edge and a first boundary line (L1) extending from a point on the first longitudinal end edge so as to be tangent to a point on the stress relaxing structure on the side of the first longitudinal end edge, and part of a second side edge section (b) extending on the side of the transverse center axis of the absorbent part perpendicular to the longitudinal center axis of the absorbent part from the intersection point of the side edge and a second bounda ry line (L2) extending from a point on the side edge of the absorbent part at which the lower end of the ear part joins to the absorbent part so as to be tangent to a point on the stress relaxing structure on the side of the transverse center axis.

2. A disposable diaper according to claim 1, wherein the point on the first longitudinal end edge from which the first boundary line extends corresponds to the intersection point of a line parallel to the longitudinal center axis of the absorbent part, and the first longitudinal end edge.

3. A disposable diaper according to claim 1, wherein the peripheral portion of the ear part is unstretchable and the stress relaxing structure is stretchable.

4. A disposable diaper according to claim 3, wherein each of the ear parts is formed of an unstretchable materi al, the stress relaxing structure is formed by processing an unstretchable material so as to be stretchable when tensioned, and the stress relaxing structure does not cause the peripheral portion of the ear part to shrink.

## Patentansprüche

1. Eine Wegwerfwindel (1), welche einen absorbierenden Teil (3) umfaßt, welcher durch Dazwischenlegen eines absorbierenden Kerns (4) zwischen ein Deckblatt (8) und ein Rückenblatt (10) gebildet ist, sodaß der absorbierende Kern dem Schritt des Trägers entsprechen kann, wenn die Wegwerfwindel am Träger angelegt ist, und welche einen ersten Längsendrand und einen zweiten Längsendrand, ein Paar Ohrteile (60, welche jeweils an der Seite des ersten Längsendrandes von den gegenüberliegenden Seitenrändern eines Längsendabschnitts des absorbierenden Teils in entgegengesetzte Richtungen abragen; und zwei Befestigungsmittel (7) aufweist, welche an den jeweiligen Seitenrändern der Ohrteile fixiert sind;
wobei jeder Ohrteil (6) eine Spannungsfreigabestruktur (20) umfaßt, in welchem eine Zugspannung, welche kleiner ist als jene, welche im peripheren Abschnitt des Ohrteils induziert ist, in einem anderen Abschnitt als in dessen peripherem Abschnitt induziert ist, dadurch gekennzeichnet, daß jedes Befestigungsmittel, welches in einem ziehenden Teilstück des Seitenrandes des Ohrteils am Ohrteil fixiert ist, am Seitenrand des Ohrteils mindestens einen Teil eines ersten Seitenrandteilstücks (a) überlappt, das sich an der Seite des ersten Längsendrandes des absorbierenden Teils vom Schnittpunkt des Seitenrandes und einer ersten Begrenzungslinie (L1), welche sich von einem Punkt am ersten Längsendrand erstreckt, um so Tangente zu einem Punkt an der Spannungsfreigabestruktur an der Seite des ersten Längsendrandes zu sein, erstreckt, und ein Teil eines zweiten Seitenrandteilstücks (b) sich an der Seite der querlaufenden Mittelachse des absorbierenden Teils lotrecht zur Längsmittelachse des absorbierenden Teils vom Schnittpunkt des Seitenrandes und einer zweiten Begrenzungslinie (L2) erstreckt, welche sich von einem Punkt am Seitenrand des absorbierenden Teils, an welchem das untere Ende des Ohrteils sich mit dem absorbierenden Teil vereinigt, erstreckt, um so Tangente zu einem Punkt an der Spannungsfreigabestruktur an der Seite der querlaufenden Mittelachse zu sein.

2. Eine Wegwerfwindel nach Anspruch 1, bei welcher der Punkt am ersten Längsendrand, von welchem sich die erste Begrenzungslinie erstreckt, dem Schnittpunkt einer Linie parallel zur längslaufenden Mittelachse des absorbierenden Teils und des ersten Längsendrandes entspricht.

3. Eine Wegwerfwindel nach Anspruch 1, bei welcher der periphere Abschnitte des Ohrteils unstreckbar ist und die Spannungsfreigabestruktur streckbar ist.

4. Eine Wegwerfwindel nach Anspuch 3, bei welcher jeder der Ohrteile aus einem unstreckbaren Material gebildet ist, die Spannungsfreigabestruktur durch Bearbeiten des unstreckbaren Materials gebildet ist, um so streckbar zu sein, wenn sie unter Zugspannung gesetzt ist, und die Spannungsfreigabestruktur den peripheren Abschnitt des Ohrteils nicht veranlaßt, zu schrumpfen.

## Revendications

1. Couche jetable (1) comprenant : une partie absorbante (3) formée par le fait d'intercaler une âme absorbante (4) entre une feuille de dessus (8) et une feuille de fond (10) de telle sorte que l'âme absorbante puisse correspondre à l'entrejambe de l'utilisateur lorsque la couche jetable est placée sur l'utilisateur, et ayant un premier bord d'extrémité longitudinal et un deuxième bord d'extrémité longitudinal ; une paire de parties d'oreille (6) faisant saillie dans des directions opposées à partir des bords latéraux opposés correspondants d'une partie d'extrémité longitudinale de la partie absorbante, du côté du premier bord d'extrémité longitudinal ; et deux moyens de fixation (7) assemblés aux bords latéraux respectifs des parties d'oreille ;
chaque partie d'oreille (6) ayant une structure à relaxation de contrainte (20), dans laquelle est induite une contrainte de tension inférieure à celle qui est induite dans la partie périphérique de la partie d'oreille, dans une partie de celle-ci autre que la partie périphérique, caractérisée en ce que chaque moyen de fixation est assemblé à la partie d'oreille dans un tronçon de traction du bord latéral de la partie d'oreille, en chevauchant au moins une partie d'un premier tronçon de bord latéral (a) sur le bord latéral de la partie d'oreille, en s'étendant sur le côté du premier bord d'extrémité longitudinal de la partie absorbante, à partir du point d'intersection du bord latéral et d'une première ligne de délimitation (L1) s'étendant à partir d'un point situé sur le premier bord d'extrémité longitudinal de façon à être tangente à un point de la structure de relaxation de contrainte, du côté du premier bord d'extrémité longitudinal, et une partie d'un deuxième tronçon de bord latéral (b) s'étendant sur le côté de l'axe central transversal de la partie absorbante, perpendiculaire à l'axe central longitudinal de la partie absorbante, à partir du point d'intersection du bord latéral et d'une deuxième ligne de délimitation (L2) s'étendant à partir d'un point situé sur le bord latéral de la partie absorbante à laquelle l'extrémité inférieure de la partie d'oreille est assemblée à la partie absorbante, de façon à être tangente à un point de la structure de relaxation de contrainte, du côté de l'axe central transversal.

2. Couche jetable selon la revendication 1, dans laquelle le point du premier bord d'extrémité longitudinal à partir duquel s'étend la première ligne de délimitation correspond au point d'intersection d'une ligne parallèle à l'axe central longitudinal de la partie absorbante, et du premier bord d'extrémité longitudinal.

3. Couche jetable selon la revendication 1, dans laquelle la partie périphérique de la partie d'oreille est non étirable et la structure de relaxation de contrainte est étirable.

4. Couche jetable selon la revendication 3, dans laquelle chacune des parties d'oreille est formée d'un matériau non étirable, la structure à relaxation de contrainte est formée par le traitement d'un matériau non étirable de façon à ce qu'il soit étirable lorsqu'il est tendu, et la structure à relaxation de contrainte n'entraîne pas la partie périphérique de la partie d'oreille à se rétrécir.
